# EUROPEAN PATENT SPECIFICATION

(11) **EP 2 869 763 B1**
(45) Date of publication and mention of the grant of the patent: **08.05.2019**
(21) Application number: 13813805.2
(22) Date of filing: 02.07.2013
(51) Int. Cl.: A61B 5/145, A61B 5/00

(54) **GLUCOSE CONSUMPTION MONITOR**
ÜBERWACHUNG DES GLUKOSEVERBRAUCHS
MONITEUR DE CONSOMMATION DE GLUCOSE

(30) Priority: 03.07.2012 US 201261667610 P; 03.07.2012 US 201261667856 P; 13.03.2013 US 201361779406 P
(43) Date of publication of application: 13.05.2015
(73) Proprietor: Edwards Lifesciences Corporation, Irvine, CA 92614 (US)
(72) Inventor: DOBBLES, John, Michael, Irvine, CA 92614 (US); KEIDAR, Yaron, Irvine, CA 92614 (US); DEPALMA, Amanda, L., Irvine, CA 92614 (US)
(74) Representative: Eisenführ Speiser
(86) International application number: PCT/US2013/049148
(87) International publication number: WO 2014/008302

(56) References cited:
- US-A1- 2005 124 872
- US-A1- 2006 009 727
- US-A1- 2009 018 426
- US-A1- 2010 168 545
- US-A1- 2012 059 237
- US-A1- 2012 065 482

## Description

### BACKGROUND

Devices for measuring various physiological parameters of a patient have been a standard part of medical care for many years. The vital signs of some patients typically are measured on a substantially continuous basis to enable physicians, nurses, and other healthcare providers to detect sudden changes in a patient's condition. Patient monitors are typically employed to display a variety of physiological patient data to physicians and other healthcare providers. Such patient data facilitates diagnosis of abnormalities or the patient's current condition.

In some circumstances, a hospital subject is continuously tested for changes in a blood analyte level, test results are evaluated by a medical professional, and a therapeutic agent is administered based on these test results. For example, of importance for health care providers with some patients is measurement of the blood glucose levels of the subject, especially in a surgical or intensive care setting. Insulin is frequently delivered in hospitals to medical patients in order to control those patients' blood glucose levels and thereby to avoid hyperglycemia.

It is important to monitor blood glucose levels over time while insulin is being administered to avoid administering the insulin too rapidly or in quantities too great, as either may result in an undesirable or even dangerous hypoglycemic condition. Glucose levels are thus evaluated regularly to ensure appropriate quantities of insulin are being delivered at appropriate rates on an appropriate schedule, in order to keep the patient's blood glucose levels within the acceptable range.

US 2006/0009727 A1 discloses a method for controlling the blood glucose level of a patient and periodically calibrating the glucose sensor using a calibration solution. The method controls the level of blood glucose in a patient through an extracorporeal blood circuit by: withdrawing blood from a vascular system in the patient to the extracorporeal circuit; removing ultrafiltrate from the withdrawn blood in the circuit; determining a level of glucose present in the blood based on the removed ultrafiltrate; infusing at least a portion of the removed ultrafiltrate and the withdrawn blood into the vascular system, and infusing insulin into the patient based on the determined level of glucose.

US 2005/0124872 A1 discloses a pulmonary artery catheter ("PAC") that is used in determining myocardial oxygen consumption. Myocardial oxygen consumption is of critical importance because decreased myocardial energy utilization during acute illness may lead to tissue hypoperfusion, multiple organ failure, and eventually death. The inventor has discovered that myocardial oxygen consumption is a function of the difference in oxygen levels in atrial and mixed venous blood. The invention has further discovered that differences in lactate, glucose or any other measurable blood concentration metabolite in atrial or superior vena cava and mixed venous blood can also be used in determining myocardial oxygen consumption.

### SUMMARY

Embodiments of the present invention provide for accurate measurement of small differences in blood glucose values at various points in the body. These accurate measurements can be used to monitor glucose uptake in specific organs of the body, for example the brain or the heart. Further, information on glucose consumption can be combined with other data, such as information on oxygen consumption, to provide an early warning of any mismatch between glucose consumption and oxygen consumption. Such an early warning could help caregivers manage insulin and sugar intake to optimize nutrition and avoid lactic acidosis. Embodiments of the invention can also optionally, automatically adjust glucose and/or insulin infusion in response to glucose consumption changes.

Embodiments of the present invention include an apparatus for monitoring consumption of glucose. In example embodiments, the apparatus includes an upstream intravascular glucose sensor configured to sense intravascular glucose representative of a first glucose concentration, and a downstream intravascular glucose sensor configured to sense intravascular glucose representative of a second glucose concentration. As an example, these sensors can be upstream and downstream of an organ in the body. In some embodiments, at least one flow control device is configured and arranged for substantially simultaneous fluid communication of a calibration fluid to multiple glucose sensors in order to reduce bias between the sensors. In some embodiments, there are multiple flow control devices; one for each glucose sensor, and a connection is provided for a common connection for a source of calibration fluid. In at least some embodiments, a monitor and control unit is connected to be in communication with the intravascular glucose sensors, and optionally, the flow control device or devices. A processor in the system is operable to calculate and optionally display at least one of a blood glucose gradient and blood glucose consumption from the first and second glucose concentrations.

In some embodiments, the monitoring apparatus includes the ability to receive input from an oxygen sensor and to calculate and optionally display at least one of an oxygen gradient and the oxygen consumption. In some embodiments, the apparatus can receive input from a carbon dioxide sensor and the calculation of the oxygen consumption is in part based on the input from the carbon dioxide sensor. In some embodiments, the monitor and control unit is operable calculate and/or display a numerical indication of a mismatch between glucose consumption and oxygen consumption. In some embodiments, the monitor and control unit is operable to make an infusion determination based on glucose consumption, estimated glucose value, or the mismatch between glucose consumption and oxygen consumption and selectively update infusion of at least one of glucose and insulin in response to the determination.

In some embodiments, the apparatus is adapted so that upstream intravascular glucose sensor is disposed in an artery upstream of an organ and the downstream intravascular glucose sensor is disposed in a vein downstream of an organ. The organ may comprise the brain or the heart.

In another aspect, the invention provides a method of monitoring glucose consumption within a patient, the method comprising calibrating an upstream intravascular glucose sensor and a downstream intravascular glucose sensor; obtaining an estimated glucose value from both the upstream intravascular glucose sensor and the downstream intravascular glucose sensor; and calculating by a processor and optionally displaying at least one of a blood glucose gradient and blood glucose consumption based on the estimated glucose values. The method may further comprise receiving input from an oxygen sensor; and calculating, by a processor, at least one of an oxygen gradient and oxygen consumption based on the input received from the oxygen sensor. The method may further comprise displaying on a display device at least one of the oxygen gradient and the oxygen consumption. The method may further comprise receiving input from a carbon dioxide sensor. In this case, the calculating of the oxygen consumption may optionally further based on the input received from the carbon dioxide sensor. The method may further comprise determining a numerical value indicative of a mismatch between glucose consumption and oxygen consumption. In this case, the method may further comprise making an infusion determination based on the numerical value; and selectively updating infusion of at least one of glucose and insulin in response to the determination. Alternatively, the method may further comprise displaying an indication of the mismatch. In this case, the upstream intravascular glucose sensor is disposed in an artery upstream of an organ and the downstream intravascular glucose sensor is disposed in a vein downstream of an organ. In this case, the organ may comprise the brain or the heart.

According to another aspect, the present invention provides a system for monitoring glucose consumption within a patient, the system comprising means for calibrating an upstream intravascular glucose sensor and a downstream intravascular glucose sensor; means for obtaining an estimated glucose value from both the upstream intravascular glucose sensor and the downstream intravascular glucose sensor; and means for calculating and optionally displaying at least one of a blood glucose gradient and blood glucose consumption based on the estimated glucose values. The system may further comprise means for receiving input from an oxygen sensor; and means for calculating at least one of an oxygen gradient and oxygen consumption based on the input received from the oxygen sensor. In this case, the system may further comprise means for receiving input from a carbon dioxide sensor. Alternatively, the system may further comprise means for determining and optionally displaying a numerical value indicative of a mismatch between glucose consumption and oxygen consumption. In this case, the system may further comprise means for selectively updating infusion of at least one of glucose and insulin in response to the means for determining.

In operation, the system calibrates the upstream and downstream intravascular sensors using the same calibration fluid to provide a common reference. Both sensor readings can be obtained through an input/output interface of a monitor and control unit. A processor in the system can calculate a blood glucose gradient from the difference in estimated glucose values from the sensors. The processor may additionally calculate glucose consumption. Oxygen and/or carbon dioxide sensor input can also be monitored and the processor can do additional calculations to determine oxygen consumption. A numerical index indicative of any mismatch, or lack thereof, between glucose consumption and oxygen consumption can be calculated and optionally displayed, and in some embodiments infusion of insulin and/or glucose into the patient can be carried out in response to the determination of the numerical value of the index or other programmatic determinations.

Embodiments of the invention can be implemented using a computer system, instruction execution platform, or a workstation with appropriate input and output capabilities connected to the appropriate glucose and oxygen or carbon dioxide sensors. Embodiments of the invention may also be implemented on a patient monitoring and control system including a display device and a processor operatively connected to the display device and the sensors and connected with a memory. The memory may be used to store present and historical numerical values as well as non-transitory computer program code which, when executed, causes the processor to carry out all or a portion of the process of an embodiment of the invention. This hardware and code form at least some of the means to carry out the various embodiments of the invention.

The invention is defined by the appended claims, the description with its examples being for illustrative purpose only.

### BRIEF DESCRIPTION OF THE DRAWINGS

FIG. 1A and FIG. IB are schematic illustrations of two-sensor calibration arrangements that can be used with embodiments of the invention to eliminate bias between sensors.
FIG. 2 is an illustration of a typical operating environment for example embodiments of the present invention.
FIG. 3 is a block diagram of a system according to example embodiments of the invention.
FIG. 4 is a flowchart illustrating a process that can be carried out with example embodiments of the invention.

### DETAILED DESCRIPTION OF EXAMPLE EMBODIMENTS

The following detailed description teaches specific example embodiments of the invention. Other embodiments do not depart from the scope of the present invention. The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms used herein should be interpreted as having a meaning that is consistent with their meaning in the context of this specification and the relevant art and will not be interpreted in an idealized or overly formal sense unless expressly so defined herein. Unless otherwise expressly stated, comparative, quantitative terms such as "less" and "more", are intended to encompass the concept of equality. As an example, "less" can mean not only "less" in the strictest mathematical sense, but also, "less than or equal to."

As will be appreciated by one of skill in the art, the present invention or portions thereof may be embodied as a method, device, article, system, computer program product, or a combination of the foregoing. Any suitable computer usable or computer readable medium may be utilized for a computer program product including non-transitory computer program code to implement all or part of an embodiment of the invention. The computer usable or computer readable medium may be, for example but not limited to, a tangible electronic, magnetic, optical, electromagnetic, or semiconductor system, apparatus or device. More specific examples (a non-exhaustive list) of the computer readable medium would include the following: a portable computer diskette, a hard disk, a random access memory (RAM), a read-only memory (ROM), an erasable programmable read-only memory (EPROM or Flash memory), a portable compact disc read-only memory (CD-ROM), or an optical storage device. The computer usable or computer readable medium may be one or more fixed disk drives or flash drives deployed in instruction execution platforms, such as servers or workstations, forming a "cloud" or network.

Computer program code to carrying out operations of the present invention or for assisting in the carrying out of a method according to an example embodiment of the invention may be written in an object oriented, scripted or unscripted programming language such as Java, Perl, Smalltalk, C++ or the like. However, the computer program code for carrying out operations of the present invention may also be written in conventional procedural programming languages, such as the "C" programming language or similar programming languages.

Computer program instructions may be provided to a processor of an instruction execution platform such as a general purpose computer, special purpose computer, server, workstation or other programmable data processing apparatus to produce a machine, such that the instructions, which execute via the processor of the computer or other programmable data processing apparatus, create means for implementing the functions/acts necessary to carry out an embodiment of the invention.

A processor used to implement an embodiment of the invention may be a general purpose digital signal processor, such as those commercially available from Texas Instruments, Inc., Analog Devices, Inc., or Freescale Semiconductor, Inc. It may also be a general purpose processor such as those typically provided for either workstation or embedded use by companies such as Advanced Micro Devices, Inc. or Intel Corporation. It could as well be a field programmable gate array (FPGA) as are available from Xilinx, Inc., Altera Corporation, or other vendors. The processor could also be a fully custom gate array or application specific integrated circuit (ASIC). Any combination of such processing elements may also be referred to as a processor, microprocessor, controller, or central processing unit (CPU). In some embodiments, firmware, software, or microcode can be stored in a non-transitory form on or in a tangible medium that is associated with the processor. Such a medium may be a memory integrated into the processor, or may be a memory chip that is addressed by the processor to perform various functions. Such firmware, software or microcode is executable by the processor and when executed, causes the processor to perform its display control and calculation functions. Such firmware or software could also be stored in or on a tangible medium such as an optical disk or traditional removable or fixed magnetic medium such as a disk drive used to load the firmware or software into a monitoring system according to embodiments of the present invention.

The term "analyte" as used herein relates to a substance or chemical constituent in a biological sample (e.g., bodily fluids, including, blood, serum, plasma, interstitial fluid, cerebral spinal fluid, lymph fluid, ocular fluid, saliva, oral fluid, urine, excretions, or exudates). Analytes can include naturally occurring substances, artificial substances, metabolites, and/or reaction products. The analyte for measurement by the sensor, devices, and methods may include glucose. Any other physiological analyte or metabolite can be substituted or combined with the measurement of glucose. The term "subject" as used herein relates to mammals, inclusive of warm-blooded animals (domesticated and non-domesticated animals), and humans.

The term "calibration" as used herein refers to one or more process of determining the relationship between sensor data and a corresponding reference data. A continuous analyte sensor can be initially calibrated, calibration can be updated or recalibrated over time (whether or not if changes in the relationship between the sensor data and reference data occur), for example, due to changes in disconnection/reconnection, sensitivity, baseline, analyte transport, metabolism, and the like. The sensed values produced by a calibrated sensor can be referred to as "calibrated values."

The phrases "operatively connected" and "operably connected" as used herein relate to one or more components linked to one or more other components, such that a function is enabled. The terms can refer to a mechanical connection, an electrical connection, or any connection that allows transmission of signals between the components. For example, one or more electrodes can be used to detect the amount of analyte in a sample and to convert that information into a signal; the signal can then be transmitted to a circuit. In such an example, the electrode is "operably connected" to the electronic circuitry. The terms include wired and wireless connections, and situations where there is are or may be intervening components.

The term "sensor" as used herein relates to a device, component, or region of a device capable of detecting and/or quantifying and/or qualifying an analyte in the intravascular and/or subcutaneous space of a subject. The phrase "sensor system" as used herein relates to a device, or combination of devices operating at least in part in a cooperative manner, that is inclusive of the sensor. In some embodiments, sensor relates to a device, component, or region of a device capable of detecting and/or quantifying and/or qualifying an analyte in the intravascular space in vivo.

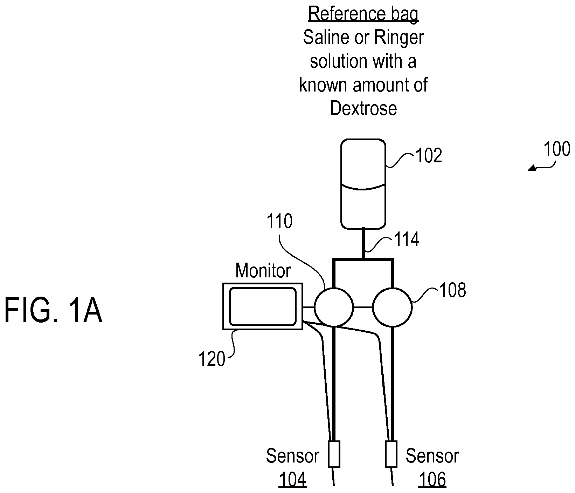

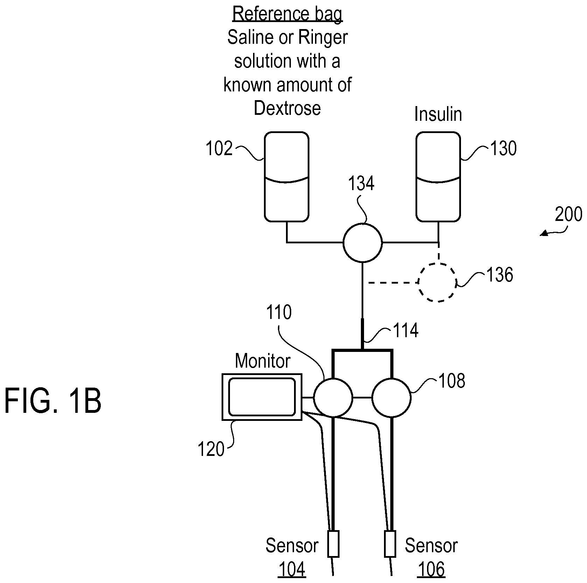

FIG. 1A is a schematic illustration of an apparatus according to example embodiments of the invention. As mentioned before, the gradient in blood glucose from arterial blood to jugular vein blood is between 0 and 10 mg/dL (it should be typically 6 mg/dL in healthy adults). That is a small gradient and any bias in the sensors used may skew the result. A way to eliminate bias between multiple sensors (two or more) according to example embodiments of the invention is to feed the sensors from the same reference calibration bag. Apparatus 100 includes a connection for a calibration bag 102 of saline or ringer solution with a known amount of dextrose. It should be noted that dextrose is a known form of glucose and that the terms may be used interchangeably herein. The bag calibrates all sensors and any error in measuring the bag itself will be eliminated when calculating the difference in blood glucose between sensors. The apparatus of FIG. 1A also includes two intravascular sensors, 104 and 106. In operation one can serve as an upstream intravascular glucose sensor configured to sense intravascular glucose representative of a first glucose concentration, such in an artery feeding an organ, and a downstream intravascular glucose sensor configured to sense intravascular glucose representative of a second glucose concentration, such as in a vein returning from the same organ. As an example, an artery and vein connected to the brain can be used. As another example, and artery and vein connected to the heart can be used.

Still referring to FIG. 1A, there are two flow control devices 108 and 110; one for each glucose sensor, and a junction 114 is provided for a common connection for the source of calibration fluid. These devices can be pumps or some other flow control devices, including those using impellers or piezoelectric elements. These interconnected items can make the entire system more compact and easy to use. Finally, a monitor 120 receives input from the sensors and may include electronics and/or firmware to control the flow control devices as well. As will be seen in the more detailed examples below, a processor-based monitor and control until can serve as the monitor, and such a device can also receive input from oxygen and/or carbon dioxide sensors.

FIG. IB illustrates an apparatus that has many of the same components as shown in FIG. 1A, as indicated by like reference numbers. The apparatus 200 of FIG. IB however includes an insulin supply 130 and at least one additional flow control device 134. In some embodiments, an additional flow control device 136 can be included. An apparatus as shown in FIG. IB can programmatically make decisions on infusing the dextrose into the patient above and beyond the calibration routine to manage the glucose status of the patient. The apparatus 200 of FIG. IB can likewise programmatically infuse insulin into the patient in response to glucose consumption determinations. The arrangement in FIG. IB may be used in a "closed-loop" or hybrid system as described herein.

Suitable intravenous sensors for use with embodiments of the invention can be configured of at least two electrodes, such as a working electrode and reference electrode. In some cases, such sensors can include three electrodes, such as but not limited to working, reference and/or counter electrodes. The sensors can be configured such that a reference electrode or counter electrode disposed remotely from the working electrode, and any combination of electrodes can be sized such that one is larger than the other electrodes. The sensors can be configured of two or more electrodes that are separated by an insulator. In some sensors, an electrode is a fine wire, such as but not limited to a wire formed from platinum, iridium, platinum-iridium, palladium, gold, silver, silver chloride, carbon, graphite, gold, conductive polymers, alloys and the like. In some exemplary embodiments, the sensors include one or more electrodes formed from a fine wire with a diameter of from about 0.001 or less to about 0.010 inches or more. The sensors can be substrate-based. Any combination of wire/substrate or two/three electrodes for the sensors are can work with embodiments of the invention. Some sensor systems that can be used with embodiments of the invention are described in U.S. Patent Publication No. 2007/0027385, the entire disclosure of which is incorporated herein by reference.

In addition to the dextrose mentioned above, a suitable reference solution (e.g., calibration solution) for use with an apparatus like that described above is saline containing a predetermined amount of dextrose (e.g., within the physiological range for a subject, or more or less than said range). In some embodiments, a baseline value of the sensor can be obtained by generating a signal when the sensor is exposed to a 0-mg/dL reference solution (or an alternative reference solution concentration). In some embodiments, updated baseline values are continuously obtained by repeatedly exposing the sensor to reference solution, e.g., in a predetermined time interval. In some embodiments, updated baseline values are continuously obtained by exposing the sensor to the reference solution for periods of time and continuously collecting baseline values. In other embodiments, such calibrations are performed "intermittently," for predetermined time intervals.

To aid in understanding of the apparatus, systems and methods illustrated herein, it may be useful to review how glucose moves through the human body. Glucose is ingested by eating and enters the circulatory system through the intestines. Glucose moves through the portal vein into the liver. Some glucose is stored. Glucose enters the circulatory system through the inferior vena cava (IVC) via the hepatic vein. Concentrated blood from the IVC is mixed with diluted blood at the right atrium, where the IVC and superior vena cava (SVC) merge. Mixed glucose travels thru the heart and lungs, entering the arterial circulation where glucose is distributed throughout the body.

Arterial blood is distributed to the upper extremities via the aortic arch and thru the subclavian arteries. Glucose is also supplied to the head and brain via the carotid arteries. An arterial sample can be pulled from the radial artery and is higher than capillary and venous blood glucose. Capillary glucose is an intermediate value, typically about 5 mg/dL lower than an arterial sample. Venous glucose can be sampled from the various veins in the forearm. Venous glucose sampled in this way is typically about 10 mg/dL lower than arterial glucose.

The proximal port of the caudal vena cava is located in the upper SVC. A sample from here combines glucose from the upper extremities and the head/brain. Such a sample should serve as a conservative as it relates directly to glucose utilization in the brain.

The system described herein uses multiple glucose sensors that can measure blood glucose simultaneously in multiple places (for example in a radial artery and a jugular vein) and display the difference in blood glucose between these sensors to calculate the rate glucose is metabolized into or out of the blood stream (for example: brain glucose consumption rate; heart glucose consumption rate). Glucose in converted to energy in the body according to the following equation:

C₆H₁₂O₆ + 6O₂ → 6CO₂ + 6H₂O.

This chemical reaction produces about 4 Kcal/g (4 Kcal of energy per 1 gram of glucose). An average human consumes about 1300 Kcal of energy per day, or 0.9 Kcal per minute. The brain is using about 20% of that energy or 0.18 Kcal/min. This equates to 45mg/min (0.18 Kcal/min divided by 4 Kcal/g) of glucose consumed. The total blood flow to the brain is about 12 mL/sec or 7.2 dL/min. In that volume the glucose consumed by the brain will result in a 6.25 mg/dL (45mg/min divided by 7.2 dL/min) difference in arterial blood to jugular vein blood.

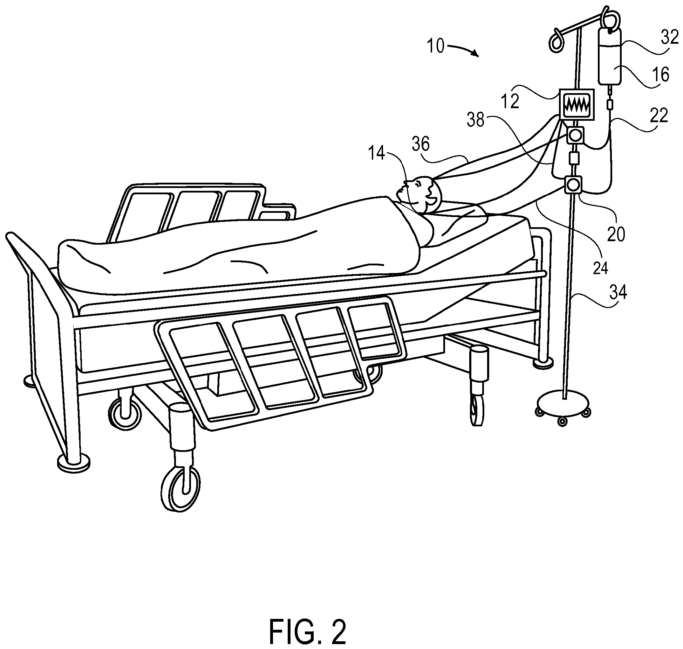

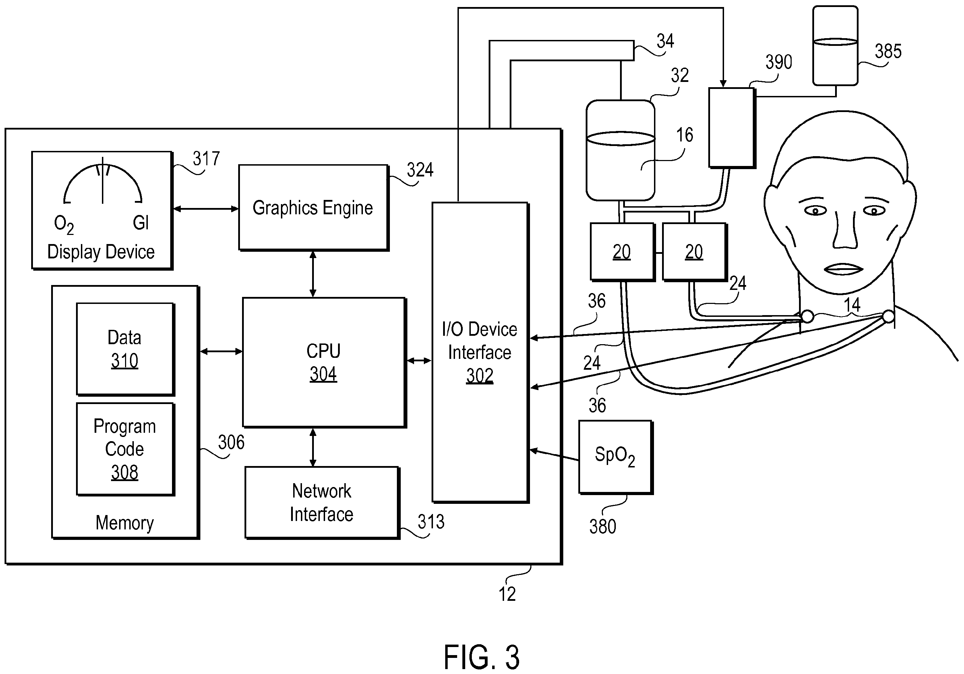

FIG. 2 and FIG. 3 depict a system that incorporates an apparatus like that of FIGs. 1A and IB. The system makes use of two sensors arranged relative to a calibration bag in the same manner as illustrated in FIGs. 1A and IB. As shown in FIG. 2, the system 10 is configured to monitor and display blood glucose levels in a hospitalized medical patient. The system is built around a monitor and control unit 12, which comprises programmed electronic circuitry to control the functioning of the system, and a display panel configured to communicate information regarding the system and its functions, as well as the condition of the patient, to a user of the system. The display panel may also serve as a touch screen interface through which a user can enter information and commands for controlling the system's operations. Access points 14 provide access for the system to the patient's body. Only one of this access points is visible in FIG. 2, however, the two access points are illustrated in FIG. 3, discussed below. The access points may provide an entry site for a catheter with a sensor placed in the vein or another vessel in the vasculature of the patient providing a reliable indicator of blood glucose levels.

FIG. 2 illustrates a configuration in which intravascular catheters are disposed inside a vein and an artery of the patient, and through which blood can be drawn over an electronic sensor to measure directly glucose levels in the patient's blood. The sensors in this configuration can be a glucose oxidase sensor as previously described configured to produce a current or voltage proportional to the patient's blood glucose level.

The system of FIG. 2 further includes a supply of calibration fluid 16 contained inside an infusion bag 32 and in fluid communication with the sensors and the access points 14 through divided fluid line 22 between the infusion bag and the two flow control devices 20 to control the flow of fluids back and forth through fluid lines 24 between the access points and the flow control devices. In this specific example, pumps are used as flow control devices. Under control of the pumps, blood may be drawn from the patient's artery or vein over each sensor. At other times, fluid may be directed from the bag to flow over and rinse a sensor, or to be infused into the patient. Various other devices can be used in place of the pumps, including piezoelectric and impeller-based devices, and any other device that can serve as a fluid controller. The infusion fluid may include normal medical saline solution with dextrose at a known concentration, which may be directed over the sensors from time-to-time in order to calibrate the sensors by reading the resultant current or voltage from the sensors at times when the known-concentration glucose solution in the infusion bag is being directed over and in contact with the sensor. Since this calibration procedure is done to both sensors at once, bias can be eliminated, as previously discussed.

The elements of system 10 are mounted on and supported by a wheeled, movable stand 34, so that the system can be moved as needed with the patient. Signal lines 36 provide electrical communication between the sensors and the monitor and control unit 12. Electrical communication is similarly provided between the monitor and control unit and the fluid pumps 20 through data and control cables 38.

The system 10 of FIG. 2 is an "open-loop" system configured to monitor the patient's blood glucose level, as determined under the control of software and circuitry of the monitor and control unit 12. The visual display and touch-screen control of the monitor and control unit communicate information including the patient's measured blood glucose level, a consumption mismatch index, and other information regarding the system's operations, to a user of the system. The user may input commands to the system through that same visual display and touch-screen control. In some embodiments, the system may programmatically and automatically determine an appropriate insulin infusion rate or dosage to be administered in the normal manner to keep the patient's blood glucose level in the appropriate range. A caregiver may observe this recommendation and act accordingly, or administer insulin at a different rate or dosage according to the care-giver's judgment and training. It should be noted that the term "system" may be used herein to refer to the entire arrangement of electronic elements and connection cables described in FIG. 2. However, the term system may also be used to refer only to the monitor and control unit including installed software and/or firmware that together direct and execute the functions described herein.

In a closed-loop system, a delivery device such as an insulin supply controller can be configured to supply insulin in a controlled fashion through an insulin supply line into the body of the patient. In a closed-loop system, the delivery of insulin to the patient can be controlled more or less automatically by the system itself, with perhaps little or no intervention by the caregiver user of the system beyond the initial setup, and with perhaps periodic checks to make sure that the system continues to function properly. Intermediate or hybrid systems also exist. Such a system combines aspects of open-loop and closed-loop systems, for example, by measuring the patient's blood glucose level, calculate a recommended or default insulin dosing level or scheme, displaying that recommendation to the user, and then await the user's input before adjusting or implementing the actual amount and timing of insulin delivery to the patient by addressing the insulin supply controller. Any of these systems may also be expected to include various alerts, alarms, and similar messages for conveying relevant information clearly to the systems' users.

FIG. 3 is an enlarged view, schematically illustrating detail of the monitor and control unit 12 of FIG. 2; however, in the case of FIG. 3, an insulin infusion arrangement has been added to create a closed-loop or hybrid system. The system includes I/O interface 302, which may in turn include an appropriate connector, and circuitry to monitor signals from the sensor system. This circuitry may include analog-to-digital converters, encoders, decoders, and the like. I/O interface device 302 is coupled to a central processing unit (CPU) 304, which controls the operation of the entire system. The I/O interface receives sensor signals and may also send signals to control the supply of insulin to the subject with some embodiments of the invention. CPU 304 is further operatively connected to memory 306. Memory 306 stores all of the information needed for the system to operate. Such information may be stored in a temporary fashion, or may be stored more permanently. This memory may include a single, or multiple types of memory. For example, a portion of the memory connected with CPU 304 may be "flash" memory, which stores information semi-permanently for use by the system. In either event memory 306 of FIG. 2 in this example embodiment includes computer program code 308 which, when executed by CPU 304, causes the system to carry out the various processes to graphically display information according to example embodiments of the invention. Memory 306 also stores data 310, which in example embodiments includes historical numerical values for the glucose consumption, oxygen consumption, and/or an index indicative of any mismatch between the two.

Still referring to FIG. 3, monitoring and control unit 12 may also include a network interface 313. This network interface can allow the system to be connected to a wired or wireless network to allow monitoring on a remote display (not shown). For example, the remote display could duplicate, or be used in place of the local display panel. Network interface 313 could also be simply used to trigger an alarm at a nurse's station or on a mobile device. In the embodiment of FIG. 3, a local display device, 317, is connected with CPU 304 via a graphics engine 324. The local display device may be an LCD panel, plasma panel, or any other type of display component and accompanying circuitry to interface the display device to graphics engine 324. Graphics engine 324 may be on its own chip, or in some embodiments it may be on the same chip as CPU 304. Note that display device 317 may include user input functionality, for example an optical or capacitive touchscreen over the display screen. In such a case, monitoring control unit 12 may include additional circuitry to process such input. Alternatively, such circuitry may be included in the display device itself, the graphics engine, or the CPU 304.

As shown in FIG. 3, the display device 317 displays an graphical indicator that is suggestive of how much, if any, mismatch there is between oxygen (O₂) consumption and glucose (Gl) consumption in the monitored patient. In order to determine the O₂ consumption, an SpO₂ sensor system 380 is connected to the I/O device interface of the monitor and control unit. This sensor system can include one or more SpO₂ sensors and may also include an SpCO₂ sensor. In response to a determination of a mismatch between glucose consumption and oxygen consumption, or to a determination of a glucose gradient, glucose consumption or even just the estimated glucose value from a sensor, CPU 304 can deliver glucose to the patent from the same reservoir used for calibration (or another reservoir), or can deliver infusion of insulin 385 using insulin flow controller 390. Insulin flow controller 390 may be a flow control device like flow control devices 20, or may be a different type of device or pump. The system thus can selectively infuse the patient with either glucose or insulin as needed. In some embodiments, the system waits for caregiver confirmation or verification before initiating or changing an infusion protocol. In some embodiments the system can be customized by the user to wait, proceed with the new protocol immediately, or wait a certain amount of time for user override before automatically proceeding. Thus, the system can programmatically provide an optimal brain physiology, e.g., during surgery, which balances the chemical energy inbound (sugar) to what the brain is actually consuming - whether the brain is hypo- or hyper-glycemic. Control of inbound brain sugar is via insulin-mediated and/or glucose infusion control of vasculature blood to the brain. Glucose can be provided from the same source used for sensor calibration as illustrated in FIG. 3, or can be provided from an additional source.

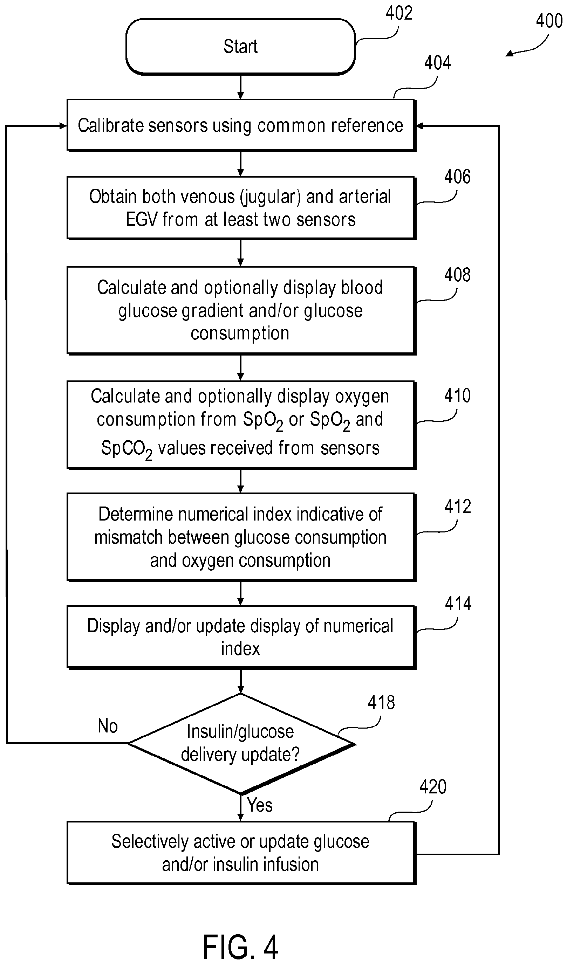

FIG. 4 is a flowchart illustrating the programmatic method of operation of the monitor and control unit included in the system illustrated in FIG. 2 and FIG. 3. Like most flowcharts, FIG. 4 illustrates process 400 as a series of subprocess blocks. The process begins at block 402. At block 404, the system calibrates the upstream and downstream intravascular sensors using the same calibration fluid to provide a common reference as previously described. Both sensor readings can be obtained at block 406 through the input/output interface of a monitor and control unit previously described. A processor in the system can calculate a blood glucose gradient at block 408 from the difference in estimated glucose values between the sensors. The processor can additionally or alternatively calculate glucose consumption. Oxygen and/or carbon dioxide sensor input can be monitored at block 410 and the processor can perform additional calculations to determine oxygen consumption. A numerical index indicative of any mismatch, or lack thereof (match), between glucose consumption and oxygen consumption can be calculated at block 412 and stored in memory. A graphical indication of the mismatch can be displayed at block 414, or the current display can be updated for the most recent calculated value. The mismatch between glucoses consumption and oxygen consumption can be evaluated based on empirical determination or historical data. The processor can also determine the mismatch by accessing a table of thresholds stored in memory.

Still referring to FIG. 4, optionally, in a closed-loop system, at block 418 a determination can be programmatically made as to whether infusion should be started, changed or updated based on the determination made at block 412. If so, either glucose or insulin infusion is selectively begun, changed or updated at block 420. For purposes of this discussion, updating the infusion of a patient can be beginning infusion, changing the rate, or switching from one infusion fluid to the other. In some embodiments, the system waits for caregiver confirmation or verification before initiating or changing an infusion protocol. In some embodiments the system can be customized by the user to wait, proceed with the new protocol immediately, or wait a certain amount of time for user override before automatically proceeding. Process 400 then continuously repeats.

A system like that described above, able to measure small differences accurately and continuously would be able to additionally be used to optimize the insulin dose as follows. If an ICU patient is hyperglycemic insulin should be administered in an infusion rate high enough to reduce blood glucose below 180 mg/dL and as close to normal (100mg/dL) as possible, but not too high as to reduce brain glucose uptake as reflected by the blood glucose gradient across the brain. In case of injury that limits blood flow or oxygen supply to the brain the target brain glucose uptake can be adjusted to a lower gradient to match the oxygen uptake and minimize anaerobic metabolism. This adjustment should minimize the brain consumption of ketones and fatty acids, and the production lactic acid. Any match or mismatch of oxygen and glucose consumption is a strong indication for treatment adjustment. A system that measures glucose gradient and oxygen consumption (by measuring SpO₂ and SpCO₂ or by measuring SpO₂ gradient) and a monitor that combines this measurements to report the degree of mismatch will provide an early warning for lactic acidosis and help optimize sugar intake, insulin management, and fluid management. A system could also be used to optimize a parenteral nutrition dose, informing that a target calorie uptake has been reached and preventing overloading the patient with sugar.

These simultaneous measurements lead to the ability to measure or estimate body glucose consumption. Such measurements or estimates may lead in turn to improved insulin dosing algorithms and to the improvement in other treatment decisions, since insulin delivery protocols might be improved if insulin were to be delivered based in part on an individual patient's ability to use that insulin effectively.

With example embodiments of the invention, one can measure blood glucose, for example, in a first blood vessel situated to deliver blood to the patient's brain, and at the same or nearly the same time, in a second blood vessel situated to carry blood away from the patient's brain. A similar arrangement can be used to measure glucose in vessels connected to the heart. An arrangement to measure glucose consumption of the brain is the arrangement of sensors illustrated in FIG. 2 and FIG. 3. In that example, one sensor is located in the jugular vein, and the other is located in one of the arteries that deliver blood to the brain as previously described. Other locations can be used, as previously described. A substantial difference in those two levels - blood glucose in the vessel upstream of the brain being higher than blood glucose downstream of the brain - would then indicate that the patient's brain was consuming that glucose effectively, and thus making good use of the insulin being delivered to the patient. A small difference, on the other hand, would indicate that the patient's brain was not using glucose effectively, thus that the current delivery of insulin was not having a good effect, and therefore that an alternative rate insulin delivery or another mode of treatment would be more appropriate. Other veins and arteries can be used for these measurements, particularly if the system is calibrated accordingly to take the distance of a sensor or sensors from the brain into account.

An additional goal of a system like that described herein is to deliver insulin to increase the patient's consumption of glucose as long as the patient is hypoglycemic. If it is determined on the other hand, that increasing the level of insulin does not increase glucose consumption, then the delivery of insulin should not be increased further, because the patient would experience no benefit from such an increase. Insulin would thus be delivered only to the extent that it would be determined currently to be having a substantial positive effect in that particular patient. Similar measurements could be made, for example, both in the patient's blood and the patient's urine, with dosing algorithms or other treatment decisions based appropriately on differences between the two measured levels, rates of change of those levels or their differences, or similar values measured by the system.

References cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification; the present specification supersedes and/or takes precedence over any such contradictory material of the incorporated reference.

All numbers expressing quantities of ingredients, reaction conditions, and so forth used in the specification are to be understood as being modified in all instances by the term "about." Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Although specific embodiments have been illustrated and described herein, those of ordinary skill in the art appreciate that any arrangement which is calculated to achieve the same purpose may be substituted for the specific embodiments shown and that the invention has other applications in other environments. This application is intended to cover any adaptations or variations of the present invention. The following claims are in no way intended to limit the scope of the invention to the specific embodiments described herein.

## Claims

1. Apparatus (100) for monitoring consumption of glucose in a patient, the apparatus (100) comprising:
an intravascular glucose sensor (104, 106) upstream of an organ of the patient and configured to sense intravascular glucose representative of a first glucose concentration;
an intravascular glucose sensor (104, 106) downstream of an organ of the patient and configured to sense intravascular glucose representative of a second glucose concentration;
**characterized by**
at least one flow control device (108, 110) configured and arranged for substantially simultaneous fluid communication of a calibration fluid (16) to both the upstream intravascular glucose sensor (104, 106) and the downstream intravascular glucose sensor (104, 106) in order to reduce bias between the upstream intravascular glucose sensor (104, 106) and the downstream intravascular glucose sensor (104, 106); and
a monitor and control unit (12) in communication with the upstream intravascular glucose sensor (104, 106) and the downstream intravascular glucose sensor (104, 106), and optionally, the at least one flow control device (108, 110), the monitor and control unit (12) operable to calculate and optionally display at least one of a blood glucose gradient and blood glucose consumption from the first and second glucose concentrations.

2. The apparatus (100) of claim 1 wherein the at least one flow control device (108, 110) further comprises:
at least two flow control devices (108, 110), one for each of the intravascular glucose sensor (104, 106) upstream of an organ of the patient and the intravascular glucose sensor (104, 106) downstream of an organ of the patient; and
a common connection (114) for a source (32) of calibration fluid (16).

3. The apparatus (100) of claim 2 wherein the monitor and control unit (12) is further operable to receive input from an oxygen sensor (380) and to calculate and optionally display at least one of an oxygen gradient and oxygen consumption.

4. The apparatus (100) of claim 3 wherein the monitor and control unit (12) is further operable to receive input from a carbon dioxide sensor (380) and wherein the calculation of the oxygen consumption is in part based on the input from the carbon dioxide sensor.

5. The apparatus (100) of claim 3 wherein the monitor and control unit (12) is further operable to display an indication of a mismatch between glucose consumption and oxygen consumption.

6. The apparatus (100) of claim 5 wherein the monitor and control unit (12) is further operable to selectively infuse at least one of glucose and insulin into the patient in response to the mismatch between glucose consumption and oxygen consumption.

7. A computer-readable medium including computer program code (308) which, when executed by a processor (304), causes the processor (304) to carry out the steps of:
simultaneously calibrating an intravascular glucose sensor (104, 106) upstream of an organ of the patient and an intravascular glucose sensor (104, 106) downstream of an organ of the patient using a common reference (16);
obtaining an estimated glucose value from both the intravascular glucose sensor (104, 106) upstream of an organ of the patient and the intravascular glucose sensor (104, 106) downstream of an organ of the patient; and
calculating by the processor (304) and optionally displaying at least one of a blood glucose gradient and blood glucose consumption based on the estimated glucose values.

8. The computer-readable medium of claim 7, further comprising computer program code (308) causing the processor (304) to carry out the steps of:
receiving input from an oxygen sensor (380); and
calculating, by the processor (304), at least one of an oxygen gradient and oxygen consumption based on the input received from the oxygen sensor (380).

9. The computer-readable medium of claim 8, further comprising computer program code (308) causing the processor (304) to carry out the step of receiving input from a carbon dioxide sensor (380), and wherein the calculating of the oxygen consumption is further based on the input received from the carbon dioxide sensor (380).

10. The computer-readable medium of claim 8, further comprising computer program code (308) causing the processor (304) to carry out the step of determining a numerical value indicative of a mismatch between glucose consumption and oxygen consumption.

11. The computer-readable medium of claim 10, further comprising computer program code (308) causing the processor (304) to carry out the steps of:
making an infusion determination based on the numerical value; and
selectively updating infusion of at least one of glucose and insulin in response to the determination.

## Patentansprüche

1. Vorrichtung (100) zur Überwachung des Glukoseverbrauchs in einem Patienten, wobei die Vorrichtung (100) enthält:
einen intravaskulären Glukosesensor (104, 106), der stromaufwärts von einem Organ des Patienten anzuordnen und dazu ausgelegt ist, intravaskuläre Glukose, die repräsentativ für eine erste Glukosekonzentration ist, zu erkennen;
einen intravaskulären Glukosesensor (104, 106), der stromabwärts von einem Organ des Patienten anzuordnen und dazu ausgelegt ist, intravaskuläre Glukose, die repräsentativ für eine zweite Glukosekonzentration ist, zu erkennen;
**gekennzeichnet durch**
zumindest eine Durchflusssteuerungseinrichtung (108, 110), die für einen im Wesentlichen gleichzeitigen Fluidaustausch eines Kalibrierungsfluids (16) mit beiden Glukosesensoren, dem stromaufwärtsseitigen intravaskulären Glukosesensor (104, 106) und dem stromabwärtsseitigen intravaskulären Glukosesensor (104, 106), ausgelegt und angeordnet ist, um die Messabweichung zwischen dem stromaufwärtsseitigen intravaskulären Glukosesensor (104, 106) und dem stromabwärtsseitigen intravaskulären Glukosesensor (104, 106) zu reduzieren; und
eine Überwachungs- und Steuerungseinheit (12), die mit dem stromaufwärtsseitigen intravaskulären Glukosesensor (104, 106) und dem stromabwärtsseitigen intravaskulären Glukosesensor (104, 106) und optional mit der zumindest einen Durchflusssteuerungseinrichtung (108, 110) verbunden ist, wobei die Überwachungs- und Steuerungseinheit (12) fähig ist, einen Blutglukosegradienten und/oder einen Blutglukoseverbrauch anhand der ersten und zweiten Glukosekonzentrationen zu berechnen und optional anzuzeigen.

2. Vorrichtung (100) nach Anspruch 1, wobei die zumindest eine Durchflusssteuerungseinrichtung (108, 110) weiter enthält:
zumindest zwei Durchflusssteuerungseinrichtungen (108, 110), eine für jeweils den intravaskulären Glukosesensor (104, 106) stromaufwärts von einem Organ des Patienten und den intravaskulären Glukosesensor (104, 106) stromabwärts von einem Organ des Patienten; und
eine gemeinsame Verbindung (114) zu einer Quelle (32) des Kalibrierungsfluids (16).

3. Vorrichtung (100) nach Anspruch 2, wobei die Überwachungs- und Steuerungseinheit (12) ferner fähig ist, einen Eingabewert von einem Sauerstoffsensor (380) zu empfangen und wenigstens einen Sauerstoffgradienten und/oder einen Sauerstoffverbrauch zu berechnen und optional anzuzeigen.

4. Vorrichtung (100) nach Anspruch 3, wobei die Überwachungs- und Steuerungseinheit (12) ferner fähig ist, einen Eingabewert von einem Kohlenstoffdioxidsensor (380) zu empfangen, und wobei die Berechnung des Sauerstoffverbrauchs teilweise auf dem Eingabewert von dem Kohlenstoffdioxidsensor basiert.

5. Vorrichtung (100) nach Anspruch 3, wobei die Überwachungs- und Steuerungseinheit (12) ferner fähig ist, ein Anzeichen für eine Diskrepanz zwischen Glukoseverbrauch und Sauerstoffverbrauch anzuzeigen.

6. Vorrichtung (100) nach Anspruch 5, wobei die Überwachungs- und Steuerungseinheit (12) ferner fähig ist, als Reaktion auf die Diskrepanz zwischen Glukoseverbrauch und Sauerstoffverbrauch wahlweise Glukose und/oder Insulin in den Patienten zu infundieren.

7. Computerlesbares Medium, das einen Computerprogrammcode (308) beinhaltet, welcher, wenn von einem Prozessor (304) ausgeführt, den Prozessor (304) dazu veranlasst, folgende Schritte auszuführen:
gleichzeitiges Kalibrieren eines intravaskulären Glukosesensors (104, 106) stromaufwärts von einem Organ des Patienten und eines intravaskulären Glukosesensors (104, 106) stromabwärts von einem Organ des Patienten unter Verwendung einer gemeinsamen Referenz (16); und
Erhalten eines geschätzten Glukosewertes von beiden Glukosesensoren, dem intravaskulären Glukosesensor (104, 106) stromaufwärts von einem Organ des Patienten und dem intravaskulären Glukosesensor (104, 106) stromabwärts von einem Organ des Patienten; und
Berechnen, durch den Prozessor (304), und optional Anzeigen eines Blutglukosegradienten und/oder eines Blutglukoseverbrauchs basierend auf den geschätzten Glukosewerten.

8. Computerlesbares Medium nach Anspruch 7, das ferner einen Computerprogrammcode (308) enthält, der den Prozessor (304) veranlasst, die folgenden Schritte auszuführen:
Erhalten eines Eingabewertes von einem Sauerstoffsensor (380); und
Berechnen, durch den Prozessor (304), eines Sauerstoffgradienten und/oder Sauerstoffverbrauchs basierend auf dem Eingabewert, der von dem Sauerstoffsensor (380) erhalten wurde.

9. Computerlesbares Medium nach Anspruch 8, das ferner einen Computerprogrammcode (308) enthält, der den Prozessor (304) veranlasst, den Schritt des Erhaltens eines Eingabewertes von einem Kohlenstoffdioxidsensor (380) auszuführen, und wobei das Berechnen des Sauerstoffverbrauchs ferner auf dem von dem Kohlenstoffdioxidsensor (380) erhaltenen Eingabewert, basiert.

10. Computerlesbares Medium nach Anspruch 8, das ferner einen Computerprogrammcode (308) enthält, der den Prozessor (304) veranlasst, den Schritt des Bestimmens eines numerischen Wertes auszuführen, der eine Diskrepanz zwischen Glukoseverbrauch und Sauerstoffverbrauch anzeigt.

11. Computerlesbares Medium nach Anspruch 10, das ferner einen Computerprogrammcode (308) enthält, der den Prozessor (304) veranlasst, die folgenden Schritte auszuführen:
Ermitteln einer Infusion basierend auf dem numerischen Wert; und
wahlweise Ändern der Infusion von Glukose und/oder Insulin als Reaktion auf die Ermittlung.

## Revendications

1. Appareil (100) pour surveiller la consommation de glucose chez un patient, l'appareil (100) comprenant :
un capteur de glucose intravasculaire (104, 106) en amont d'un organe du patient et configuré pour détecter un glucose intravasculaire représentatif d'une première concentration de glucose ;
un capteur de glucose intravasculaire (104, 106) en aval d'un organe du patient et configuré pour détecter un glucose intravasculaire représentatif d'une seconde concentration de glucose ;
**caractérisé par**
au moins un dispositif de commande d'écoulement (108, 110) configuré et agencé pour une communication fluidique sensiblement simultanée d'un fluide d'étalonnage (16) à la fois au capteur de glucose intravasculaire en amont (104, 106) et au capteur de glucose intravasculaire en aval (104, 106) afin de réduire la polarisation entre le capteur de glucose intravasculaire en amont (104, 106) et le capteur de glucose intravasculaire en aval (104, 106) ; et
une unité de surveillance et de commande (12) en communication avec le capteur de glucose intravasculaire en amont (104, 106) et le capteur de glucose intravasculaire en aval (104, 106), et éventuellement, l'au moins un dispositif de commande d'écoulement (108, 110), l'unité de surveillance et de commande (12) utilisable pour calculer et éventuellement afficher au moins l'un d'un gradient de glucose sanguin et de la consommation de glucose dans le sang à partir des première et seconde concentrations de glucose.

2. Appareil (100) selon la revendication 1, dans lequel l'au moins un dispositif de commande d'écoulement (108, 110) comprend, en outre :
au moins deux dispositifs de commande d'écoulement (108, 110), un pour chacun du capteur de glucose intravasculaire (104, 106) en amont d'un organe du patient et du capteur de glucose intravasculaire (104, 106) en aval d'un organe du patient ; et
une connexion commune (114) pour une source (32) de fluide d'étalonnage (16).

3. Appareil (100) selon la revendication 2 dans lequel l'unité de surveillance et de commande (12) est en outre utilisable pour recevoir une entrée provenant d'un capteur d'oxygène (380) et pour calculer et éventuellement afficher au moins l'un parmi un gradient d'oxygène et une consommation d'oxygène.

4. Appareil (100) selon la revendication 3 dans lequel l'unité de surveillance et de commande (12) est en outre utilisable pour recevoir une entrée provenant d'un capteur de dioxyde de carbone (380) et dans lequel le calcul de la consommation d'oxygène est en partie basé sur l'entrée provenant du capteur de dioxyde de carbone.

5. Appareil (100) selon la revendication 3, dans lequel l'unité de surveillance et de commande (12) est en outre utilisable pour afficher une indication d'une disparité entre la consommation de glucose et la consommation d'oxygène.

6. Appareil (100) selon la revendication 5 dans lequel l'unité de surveillance et de commande (12) est en outre utilisable pour perfuser sélectivement au moins l'un parmi le glucose et l'insuline dans le patient en réponse à la disparité entre la consommation de glucose et la consommation d'oxygène.

7. Support lisible par ordinateur comprenant un code de programme informatique (308) qui, lorsqu'il est exécuté par un processeur (304), amène le processeur (304) à mettre en oeuvre les étapes consistant à :
étalonner simultanément un capteur de glucose intravasculaire (104, 106) en amont d'un organe du patient et un capteur de glucose intravasculaire (104, 106) en aval d'un organe du patient à l'aide d'une référence commune (16) ;
obtenir une valeur de glucose estimée à partir à la fois du capteur de glucose intravasculaire (104, 106) en amont d'un organe du patient et du capteur de glucose intravasculaire (104, 106) en aval d'un organe du patient ; et
calculer par le processeur (304) et éventuellement à afficher au moins l'un d'un gradient de glucose sanguin et d'une consommation de glucose dans le sang sur la base des valeurs de glucose estimées.

8. Support lisible par ordinateur selon la revendication 7 comprenant en outre un code de programme informatique (308) amenant le processeur (304) à mettre en oeuvre les étapes consistant à :
recevoir une entrée provenant d'un capteur d'oxygène (380) ; et
à calculer par le processeur (304), au moins l'un parmi un gradient d'oxygène et une consommation d'oxygène sur la base de l'entrée reçue du capteur d'oxygène (380).

9. Support lisible par ordinateur selon la revendication 8, comprenant en outre un code de programme informatique (308) amenant le processeur (304) à mettre en oeuvre l'étape de réception d'entrée à partir d'un capteur de dioxyde de carbone (380), et dans lequel le calcul de la consommation d'oxygène est en outre basé sur l'entrée reçue du capteur de dioxyde de carbone (380).

10. Support lisible par ordinateur selon la revendication 8 comprenant en outre un code de programme informatique (308) amenant le processeur (304) à effectuer l'étape de détermination d'une valeur numérique indicative d'une disparité entre la consommation de glucose et la consommation d'oxygène.

11. Support lisible par ordinateur selon la revendication 10 comprenant en outre un code de programme informatique (308) amenant le processeur (304) à mettre en oeuvre les étapes consistant à :
réaliser la détermination d'une perfusion sur la base de la valeur numériqu ; et
à mettre à jour sélectivement une perfusion d'au moins un parmi le glucose et l'insuline en réponse à la détermination.
